# FASCICULE DE BREVET EUROPEEN

(11) **EP 3 457 842 B1**
(45) Date de publication et mention de la délivrance du brevet: **25.03.2020**
(21) Numéro de dépôt: 17723123.0
(22) Date de dépôt: 15.05.2017
(51) Int. Cl.: A01N 1/02, G01N 1/42, G01N 35/00

(54) **INSTALLATION DE MANIPULATION ET STOCKAGE D'ECHANTILLONS BIOLOGIQUES A TRES BASSES TEMPERATURES**
ANLAGE ZUR HANDHABUNG UND LAGERUNG VON BIOLOGISCHEN PROBEN BEI SEHR NIEDRIGEN TEMPERATUREN
FACILITY FOR HANDLING AND STORING BIOLOGICAL SAMPLES AT VERY LOW TEMPERATURES

(30) Priorité: 17.05.2016 FR 1654373
(43) Date de publication de la demande: 27.03.2019
(73) Titulaire: Irelec, 38400 Saint-Martin-D'Heres (FR)
(72) Inventeur: PRIVAT DE FORTUNE, Matthieu, 38400 Saint-Martin-D'Heres (FR); MAROT, Gérard, 38330 Montbonnot-Saint-Martin (FR); CUNRATH, Aymeric, 38920 Crolles (FR); BRIAND, Yohan, 38500 Saint-Nicolas-de-Macherin (FR); TANG, Rattena, 38000 Grenoble (FR)
(74) Mandataire: Nony
(86) Numéro de dépôt international: PCT/EP2017/061632
(87) Numéro de publication internationale: WO 2017/198628

(56) Documents cités:
- EP-A2- 2 146 163
- EP-A2- 2 169 405
- EP-A2- 2 492 663
- WO-A1-2011/047710
- KR-A- 20140 099 810
- US-A1- 2012 283 867

## Description

### Domaine technique

La présente invention concerne le domaine des installations usuellement appelées « bio-banques », qui permettent le stockage d'échantillons biologiques à très basses températures et notamment dans des conditions cryogéniques, grâce en particulier à de l'azote liquide en tant que source de froid.

Elle a trait plus particulièrement aux bio-banques automatisées, c'est-à-dire dans lesquelles la manipulation des échantillons stockés est réalisée sans l'intervention d'un opérateur.

L'invention vise plus particulièrement à améliorer ce type de bio-banques, notamment en permettant d'utiliser les récipients de stockage des échantillons déjà présents dans le commerce.

Bien que décrite en référence à une application préférée d'utilisation de récipients de stockage cryogéniques du commerce, l'invention peut s'appliquer à tous types de récipients de stockage.

En outre, bien que décrite en référence à une application préférée dans laquelle les échantillons biologiques sont contenus dans des tubes de conservation, l'invention peut aussi s'appliquer à tous types de conteneurs d'échantillons biologiques, notamment à des poches de sang.

### Art antérieur

Dans les bio-banques conventionnelles, les conteneurs d'échantillons biologiques sont stockés dans des récipients refroidis par de l'azote liquide, typiquement dans une gamme de températures allant de -150°C à -200°C, et leur manipulation, c'est-à-dire leur chargement/déchargement d'un récipient est assurée manuellement par un opérateur. Les avantages de telles bio-banques sont le coût d'investissement minimal, une flexibilité d'utilisation avec peu de maintenance à réaliser.

Cependant, elles présentent des inconvénients importants dont la gestion des échantillons qui se fait par définition manuellement avec les risques d'erreur de manipulation afférents, la traçabilité des échantillons qui n'est sûre à 100% et enfin, pour les opérateurs, les risques d'anoxie liés à l'environnement cryogénique (présence d'azote liquide), le manque d'ergonomie et le niveau d'efforts à fournir pour soulever les rangements depuis un récipient de stockage (poids important, phénomène d'adhésion dû au froid).

Différentes tentatives d'automatisation de bio-banques ont déjà été réalisées.

On peut citer les demandes de brevet WO2014001184 A1, EP2232175 A2, CN102269667 A, US2012309297 A1, JP2002205804 A, EP1012515 A1 qui divulguent des solutions de bio-banques automatisées.

Si l'automatisation amène des avantages conséquents comme la garantie de traçabilité des échantillons sur base de données, la sécurité des opérateurs et des opérations de manipulation, les bio-banques automatisées déjà proposées présentent des inconvénients majeurs.

Tout d'abord, elles nécessitent un investissement très important à l'achat comme en fonctionnement et en maintenance, du fait d'une conception sur mesure pour chaque configuration, avec notamment l'impossibilité de réutiliser les équipements standard déjà existants, comme les récipients de stockage standard.

Ensuite, leur conception fait que leur gamme de température est restreinte, avec pour bon nombre l'impossibilité d'atteindre des températures cryogéniques typiquement inférieures à -150°C.

Enfin, comme évoqué, leur conception sur mesure fait qu'elles sont dédiées à une seule et unique configuration, ce qui ne permet pas de les faire évoluer.

EP 2 169 405 A2 divulgue un stockage robotique pour stocker des microplaques portant une pluralité de tubes d'échantillons biologiques, comprenant une chambre de congélation ayant une pluralité des congélateurs et un premier robot étant adapté de retirer une microplaque et de placer une microplaque dans un congélateur. Les microplaques sont stockées dans un congélateur de sorte qu'une pluralité des microplaques soient disposées l'une au-dessus l'autre dans un réceptable pour microplaques. Le stockage comprend une chambre de traitement comprenant une station de traitement ayant au moins un module de transfer de tubes ainsi qu'un deuxième robot pour déplacer les microplaques entre les réceptables de microplaques et le module de transfer de tubes. Les réceptables de microplaques comprennent des "stackers" et / ou des hôtels de stockage.

Il existe donc un besoin d'améliorer les installations de manipulation et de stockage d'échantillons biologiques, ou bio-banques et ce notamment en s'affranchissant des inconvénients à la fois des bio-banques conventionnelles dans lesquelles la manipulation des échantillons est assurée manuellement et des bio-banques automatisées déjà proposées.

Le but de l'invention est de répondre au moins en partie à ce besoin.

### Exposé de l'invention

Pour ce faire, l'invention concerne une installation de manipulation et de stockage d'échantillons biologiques à très basses températures, notamment dans des conditions cryogéniques comprenant :
- une pluralité de boites de rangement à compartiments multiples, chaque compartiment étant adapté pour loger un conteneur adapté chacun pour contenir un ou des échantillons biologiques ;
- une pluralité de colonnes de rangement, chacune s'étendant selon un axe longitudinal et divisée en une pluralité de tiroirs de rangement, dits racks, adaptés chacun pour recevoir par translation transversale à l'axe longitudinal une de la pluralité des boites ;
- au moins un récipient de stockage, à isolation thermique, dont l'intérieur est apte à être soumis à de très basses températures, comprenant dans sa partie supérieure une grille alvéolée dont chaque alvéole est adaptée pour recevoir à la verticale une de la pluralité des colonnes;
- un poste de transfert comprenant une table de transfert sur laquelle un opérateur peut amener une ou plusieurs boite(s) à compartiments multiples, dite boite de transfert, dont chaque compartiment est également adapté pour loger un d'une pluralité de conteneurs adaptés chacun pour contenir un ou des échantillons biologiques;
- un poste de chargement/déchargement des boites de rangement à l'unité, comprenant une table, dite table d'attente, au moins un moyen d'extraction pour extraire n'importe laquelle des boites de rangement, depuis un rack d'une colonne positionnée dans ledit poste et l'amener jusqu'à une position posée sur la table d'attente et au moins un moyen de poussée pour pousser une boite, depuis sa position posée sur la table d'attente, jusque dans un rack vide d'une colonne positionnée dans ledit poste ;
- un poste de chargement/déchargement des conteneurs à l'unité, comprenant une table, dite table de manipulation, adaptée pour supporter au moins deux boites à compartiments, dont l'une est la boite de transfert et l'autre est l'une des boites de rangement sélectionnée;
- au moins un robot comprenant un organe de préhension adapté pour réaliser la préhension soit de l'extrémité supérieure d'une colonne de rangement, soit d'une boite de rangement, soit d'un conteneur ; le(s) robot(s) étant adapté(s) pour permettre un mouvement du ou des organes de préhension selon six degrés de liberté.

Selon l'invention, l'installation est configurée de sorte que l'organe de préhension du robot puisse à la fois :
i/ amener la boite de transfert depuis la table de transfert sur la table de manipulation,
ii/ extraire n'importe laquelle des colonnes depuis une des alvéoles de la grille du récipient de stockage puis l'amener dans le poste de chargement/déchargement des boites dans une position telle que le moyen d'extraction puisse extraire une boite de rangement sélectionnée sur la table d'attente,
iii/amener la boite de rangement sélectionnée depuis la table d'attente sur la table de manipulation,
iv/ extraire à l'unité n'importe lequel des conteneurs depuis un des compartiments de la boite de rangement sélectionnée, positionnée sur la table de manipulation et l'amener dans un des compartiments de la boite de transfert,
et vice-versa avec le moyen de poussée pour pousser une boite de rangement sélectionnée dans un rack vide d'une colonne positionnée dans le poste de chargement/déchargement des boites.

L'expression « vice-versa » signifie ici que les étapes i/ à iv/ mises en œuvre par le robot six axes et son organe de préhension sont réalisées dans l'autre sens du chargement/déchargement au transfert, seul le moyen de poussée agit en lieu et place du moyen d'extraction (étape ii).

Par « très basses températures », on entend ici et dans le cadre de l'invention, des températures inférieures à -100°C, de préférence inférieures à -150 °C.

Selon un mode de réalisation avantageux, l'installation comprend un seul robot à six degrés de liberté de mouvement, dit robot six axes.

Ainsi, l'invention consiste essentiellement à utiliser et agencer judicieusement au moins un robot, à six axes de déplacement relativement à un ou des récipients de stockage et un poste de transfert des conteneurs à l'unité de sorte à mettre à profit la flexibilité de déplacement et de préhension offerte par le(s) bras du(des) robot(s) et de son (leur) organe de préhension afin d'automatiser l'ensembles des opérations faites manuellement par un opérateur dans les bio-banques classiques.

Ainsi, le(s) bras du(des) robot(s) avec son(leur) organe de préhension effectue à lui seul les opérations qui sont réalisées usuellement à la main dans les bio-banques selon l'état de l'art. Ces opérations peuvent être résumées comme suit :
- extraction et insertion des boites de rangement hors et dans le poste de transfert ;
- extraction et insertion des colonnes de rangement hors et dans les récipients de stockage ;
- extraction et insertion des boites de rangement hors et dans les colonnes ;
- extraction et insertion des conteneurs d'échantillons biologiques hors et dans les boites.

De manière surprenante, bien que de nombreuses tentatives d'automatisation des bio-banques ont déjà été faites par le passé, aucune n'a pensé à mettre à profit la flexibilité de déplacement offerte par le bras d'un robot six axes et son organe de préhension.

Les avantages de l'installation selon l'invention sont nombreux parmi lesquels on peut citer :
- coût inférieur aux bio-banques automatisées selon l'état de l'art qui sont nécessairement spécifiques tout en conservant leurs avantages inhérents que sont l'automatisation, la sécurité et la traçabilité des échantillons (lecture code barre, base de données...), la sécurité des opérateurs ;
- possibilité d'implanter l'ensemble des composants de l'installation dans une enceinte à environnement contrôlé, ce qui garantit l'intégrité des échantillons (température, givre, ...);
- réutilisation possible des récipients de stockage existants dans les bio-banques conventionnelles, notamment les récipients fonctionnant dans des conditions cryogéniques ;
- modularité d'implantation, avec en particulier la possibilité de mettre plus ou moins de récipients de stockage, de déplacer le robot sur un rail pour avoir accès à un très grand nombre de récipients de stockage alignés.
- possibilité de pouvoir continuer l'exploitation de l'installation de manière manuelle, ce qui permet d'assurer le fonctionnement continu tout en facilitant les opérations de maintenance, et conserver une certaine flexibilité.
- possibilité d'évolution de l'installation ;
- facilité et rapidité d'automatisation d'une bio-banque conventionnelle déjà exploitée.

Selon un mode de réalisation avantageux, l'installation comprend une enceinte à environnement contrôlé comprenant un accès sécurisé au poste de transfert pour un opérateur depuis l'extérieur, l'enceinte étant configurée pour loger la pluralité des boites de rangement, la pluralité des colonnes de rangement, le(s) récipient(s) de stockage, le poste de transfert, les postes de chargement/déchargement et le robot six axes.

L'accès au poste de transfert peut consister en un sas étanche ou en un tiroir étanche.

Afin de garantir la traçabilité, chaque colonne et/ou chaque boite de rangement et/ou la boite de transfert peu(ven)t comprendre avantageusement un moyen d'identification, de préférence un code à barres (uni ou bidimensionnel), par un lecteur d'identification porté par le robot.

Grâce à la flexibilité de déplacement et de préhension du robot, plusieurs configurations d'implantation de l'installation peuvent être envisagées, notamment les configurations avantageuses suivantes :
- une pluralité de récipients de stockage agencés en formant sensiblement une portion de cercle avec le robot fixé et agencé à l'intérieur de la portion de cercle, le poste de chargement/déchargement des boites à l'unité agencé à l'intérieur du cercle, le poste de chargement/déchargement des conteneurs à l'unité agencé sur le cercle ;
- une pluralité de récipients de stockage agencés en formant sensiblement au moins une rangée, avec le robot monté sur un chariot mobile sur un rail le long de la rangée.

Selon une variante avantageuse, le ou les récipients de stockage comprennent chacun un couvercle pour fermer le récipient ainsi en configuration de stockage, non concerné par l'extraction depuis ou réciproquement l'insertion d'une colonne en son sein.

Selon cette variante, l'unité de contrôle-commande du robot est de préférence adaptée pour commander l'ouverture et réciproquement la fermeture de chaque récipient de stockage.

Avantageusement, on peut prévoir un ou plusieurs vérins, en tant que moyen(s) d'extraction et/ou moyen(s) de poussée des boites d'une colonne lorsqu'elle est positionnée sur la table d'attente,
Selon une variante avantageuse, on peut prévoir en tant que moyens d'extraction, plusieurs vérins positionnés chacun en regard d'un rack d'une colonne de rangement, lorsqu'elle est positionnée sur la table de manipulation.

L'organe de préhension du robot peut également constituer en lieu et place de vérin(s), le moyen de poussée.

Les boites de rangement et de transfert sont avantageusement adaptées pour loger des tubes de conservation ou des enveloppes souples, telles que des poches de sang en tant que conteneurs d'échantillons biologiques.

Les boites de rangement peuvent comprendre chacune un couvercle, l'organe de préhension du robot étant alors adapté pour ôter un couvercle de sa boite avant l'extraction à l'unité des conteneurs logés dans ladite boite, et vice-versa.

De préférence, le ou les récipients de stockage fonctionnent à des conditions cryogéniques, notamment par azote liquide en tant que source de froid.

Selon un mode de réalisation avantageux, le poste de chargement/déchargement des conteneurs à l'unité est configuré en enceinte isolée à atmosphère contrôlée à de très basses températures. Cela permet d'assurer une protection efficace contre le givre par une atmosphère sèche et un maintien en froid pour les échantillons biologiques.

L'invention a également pour objet l'utilisation de l'installation décrite précédemment, pour stocker des échantillons biologiques dans des conditions cryogéniques sous enceinte à environnement contrôlé.

### Description détaillée

D'autres avantages et caractéristiques de l'invention ressortiront mieux à la lecture de la description détaillée d'exemples de mise en œuvre de l'invention faite à titre illustratif et non limitatif en référence aux figures suivantes parmi lesquelles :
- la figure 1 est une vue en perspective d'un exemple d'une partie d'installation de manipulation et de stockage d'échantillons biologique selon l'invention;
- la figure 1A est une vue de détail en perspective de la figure 1 montrant le poste de transfert depuis l'extérieur de l'installation;
- la figure 2 est une vue de dessus de la figure 1;
- la figure 3 est une vue en perspective montrant le poste de chargement/déchargement des boites de rangement à l'unité de l'installation selon l'invention, avec une colonne de rangement des boites en position dans le poste;
- la figure 4 est une vue en perspective montrant l'organe de préhension du robot six axes de l'installation selon l'invention;
- la figure 5 est une vue en perspective d'un poste de chargement/déchargement des conteneurs d'échantillons à l'unité, de l'installation selon l'invention;
- la figure 6 est une vue en perspective d'un tube de conservation d'échantillons biologiques convenant en tant que conteneur convenant à l'installation selon l'invention.

On précise ici dans l'ensemble de la présente demande, les termes « inférieure », « centrale », « supérieure », « dessus », « dessous », « intérieure », « extérieure», sont à comprendre par référence à un récipient de stockage et une colonne de rangement de l'installation selon l'invention agencée à la verticale.

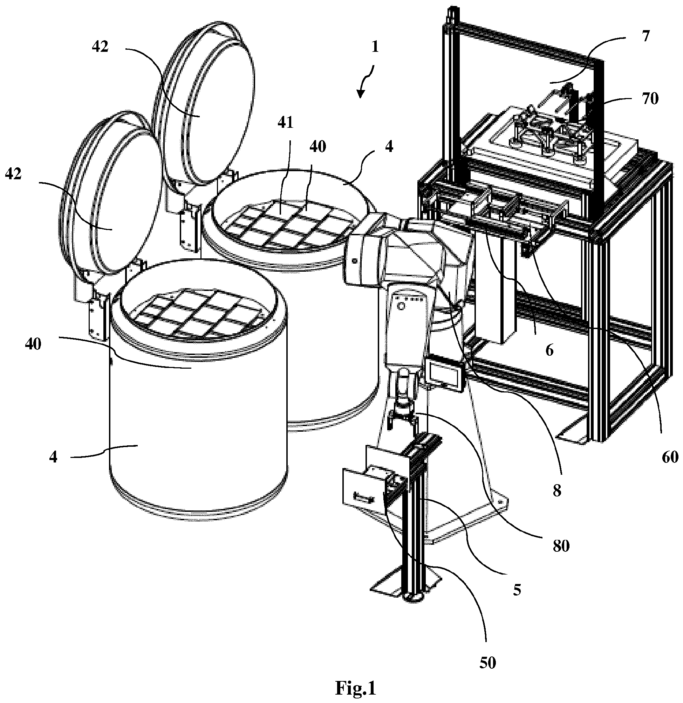

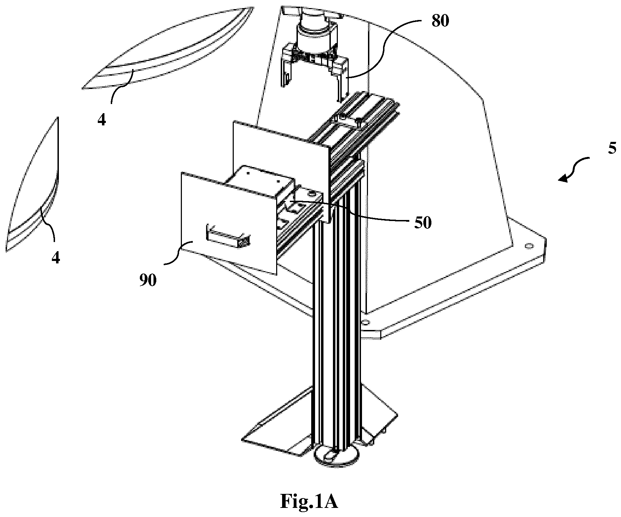

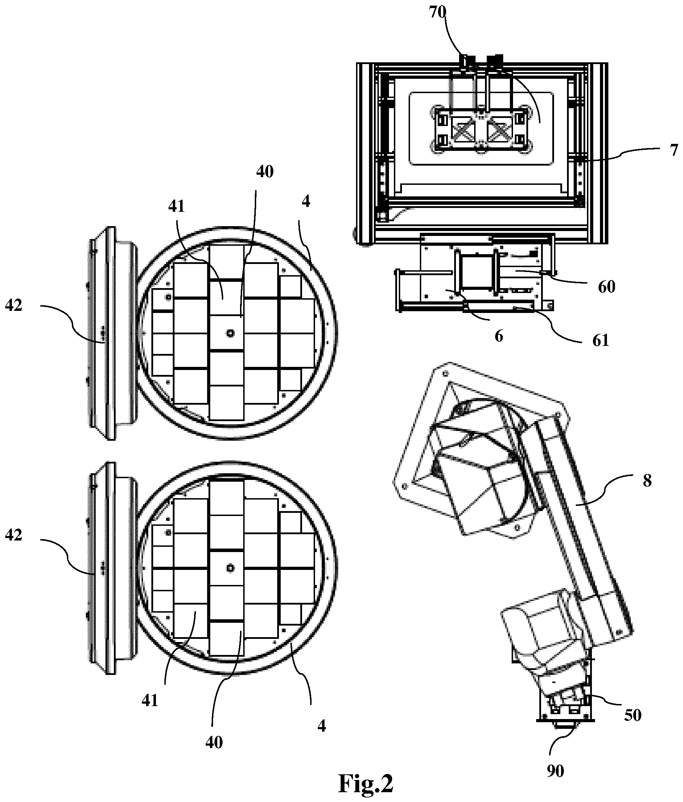

On a représenté en figures 1 et 2, l'ensemble des composants essentiels d'une installation 1 de manipulation et de stockage d'échantillons biologiques à très basses températures, notamment dans des conditions cryogéniques, selon l'invention.

Les échantillons biologiques sont contenus dans des conteneurs.

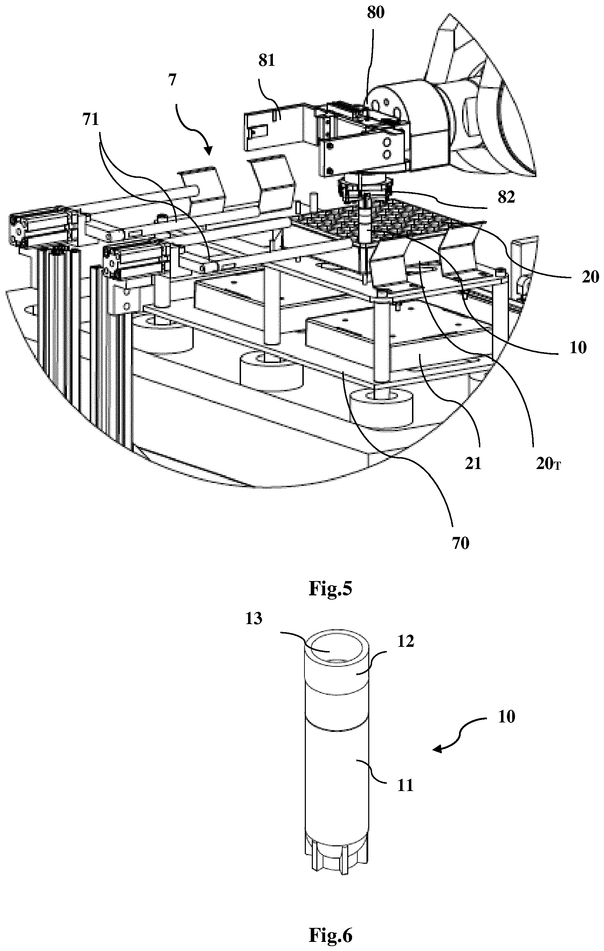

Dans l'exemple illustré, les conteneurs d'échantillons 10 mis en œuvre sont des tubes de conservation déjà utilisés. Un tube de conservation 10 est illustré en figure 6 : il comprend une partie tube borgne 11 sur laquelle un bouchon 12 avec creux intérieur 13 est fixé de manière amovible.

Une pluralité de tubes 10 est logée individuellement dans le compartiment 20 d'une boite 2 de rangement à compartiments multiples. Le logement d'un tube 10 dans un compartiment 20 est illustré en figure 5. Sur cette figure 5, on distingue en outre un couvercle 21 de boite 2 qui est ôté de celle-ci afin de permettre l'extraction ou l'insertion à l'unité des tubes 10 dans les compartiments 20 de la boite 2.

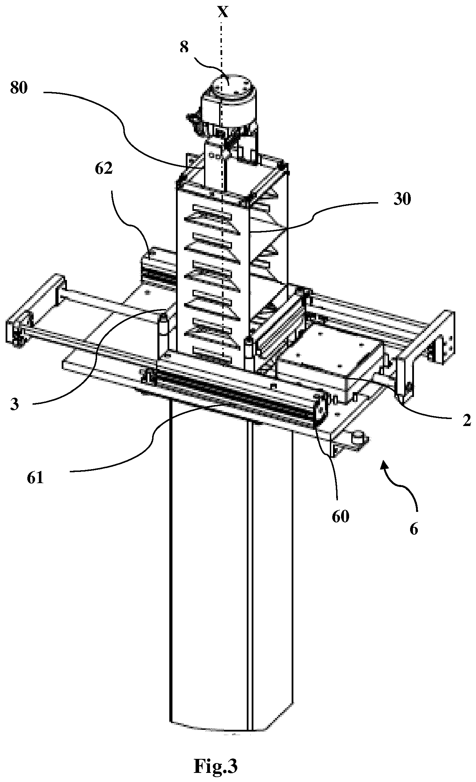

L'installation 1 comprend des colonnes de rangement 3 à tiroirs ou racks. Comme montré en figure 3, une colonne de rangement 3, s'étend selon un axe longitudinal X et est divisée en une pluralité de racks 30 de rangement. Chaque rack 30 peut recevoir par translation transversale à l'axe longitudinal X, une boite de rangement 2.

Deux récipients de stockage 4 identiques, à isolation thermique, dont l'intérieur est apte à être soumis à de très basses températures, notamment par de l'azote liquide en tant que source de froid sont agencés. Le récipient peut être de type à double-paroi dans laquelle le vide est réalisé pour garantir l'isolation thermique avec l'extérieur. De préférence, les récipients 4 sont fixés au sol.

Chaque récipient 4 comprend dans sa partie supérieure une grille alvéolée 40 dont chaque alvéole 41 peut recevoir à la verticale une colonne de rangement 3. Chaque récipient 4 est fermé par un couvercle amovible 42.

L'installation 1 selon l'invention comporte une enceinte à environnement contrôlé, non représentée, dans laquelle tous les composants essentiels sont logés.

Pour assurer le transfert à l'unité des boites de rangement 2 depuis l'extérieur de l'enceinte, il est implanté un poste de transfert 5 comprenant une table de transfert 50 à laquelle un opérateur peut avoir accès et y amener une boite de transfert 2T aux compartiments 20 identiques à ceux des autres boites. De préférence, l'accès est réalisé par un système de sas ou de tiroir isolé et à atmosphère contrôlée qui permet de garantir la sécurité de l'opérateur.

L'installation comprend également un poste de chargement/déchargement 6 des boites 2 à l'unité, qui est un poste intermédiaire entre les récipients de stockage 4 et un poste de chargement/déchargement 7 des conteneurs à l'unité.

Comme illustré en figure 3, le poste 6 est muni d'une table d'attente 60 avec sur chacun de ses bords longitudinaux un vérin 61 ou 62. L'un des vérins 61 permet d'extraire n'importe laquelle des boites de rangement 2, depuis un rack 30 d'une colonne 3 positionnée dans ledit poste 6 et l'amener jusqu'à une position posée sur la table d'attente 60. L'autre vérin 62 permet de pousser une boite 2, depuis sa position posée sur la table d'attente 60, jusque dans un rack vide 30 d'une colonne 3 positionnée dans ledit poste 6.

Pour assurer le chargement/déchargement 7 des tubes d'échantillons 10 à l'unité, un poste 7 est agencé à proximité du poste 6. Comme illustré en figure 5, ce poste 7 comprend une table de manipulation 70, adaptée pour supporter au moins deux boites 2, dont l'une est la boite de transfert 2_{T} et l'autre est une des boites de rangement sélectionnée. Ce poste 7 est avantageusement configuré en environnement froid contrôlé.

Enfin, au centre de l'installation 1, un robot 8 six axes est fixé au sol avec un bras sur lequel est monté un organe de préhension 80, 81, 82. Ce dernier est adapté pour réaliser à la préhension soit de l'extrémité supérieure d'une colonne de rangement 3, soit d'une boite de rangement 2, soit d'un tube d'échantillons 10.

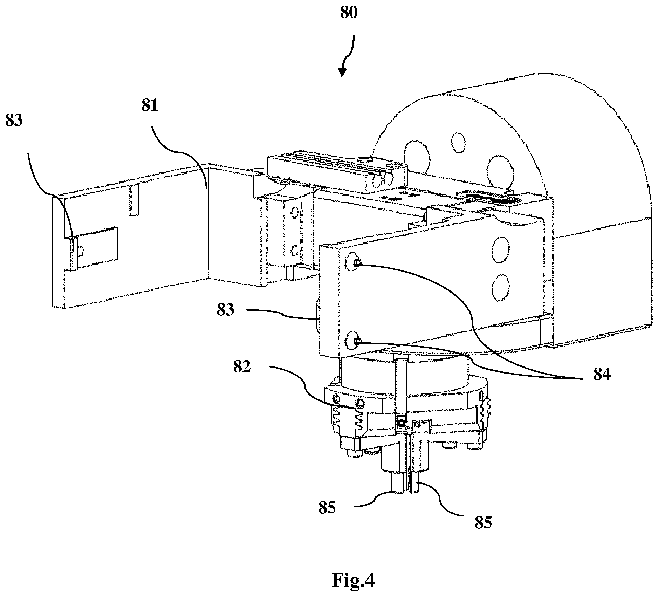

L'organe de préhension 80 est détaillé en figure 4 : il comprend une pince 81 à deux branches mobiles l'une par rapport à l'autre, et un élément de préhension 82 des tubes 10 à l'unité monté fixe et à 90° de l'axe de la pince 81.

La pince 81 supporte à l'intérieur de chacune de ses branches, une cale de préhension 83 et à l'extérieur de chacune de ses branches des ergots de préhension 84.

L'élément de préhension 82 comprend quant à lui, un ou plusieurs doigts de préhension 85, avantageusement trois concentriques et disposés à 120° l'un de l'autre.

Le fonctionnement de l'organe de préhension est le suivant :
- pour assurer la préhension d'une colonne de rangement 3, les branches de la pince 81 sont écartées l'une de l'autre, les ergots de préhension 84 viennent alors se verrouiller dans des creux correspondants dans la partie supérieure de la colonne ;
- pour assurer la préhension d'une boite 2, les branches de la pince 81 sont rapprochées mutuellement, les cales de préhension 83 viennent alors en appui contre le fond de la boite tandis que les branches viennent en appui sur les côtés latéraux de la boite ;
- pour assurer la préhension d'un tube 10, les doigts 85 rapprochés mutuellement s'insèrent dans le creux 13 du bouchon 12 du tube puis sont écartés les uns des autres de sorte à être verrouillés avec le bouchon 12.

On décrit maintenant les différentes étapes de fonctionnement de l'installation 1 selon l'invention mis en œuvre par une unité de contrôle-commande du robot 8 et des récipients de stockage 4, respectivement pour une opération de retrait de leur stockage d'un ou plusieurs tubes d'échantillons vers l'extérieur de l'enceinte de l'installation et à l'inverse, pour le dépôt d'un ou plusieurs tubes d'échantillons depuis l'extérieur de l'enceinte, dans un récipient de stockage.

### 1/ Retrait

Un opérateur sélectionne, par l'intermédiaire d'une interface homme-machine le retrait d'un tube d'échantillons 10.

L'unité de contrôle vérifie alors dans sa base de données que la colonne 3 et la boite de rangement 2 et le tube d'échantillons 10 sélectionné sont bien présents.

Si la réponse est positive, l'opérateur dépose alors dans le sas ou le tiroir étanche d'accès 90, une boite de transfert 2_{T} à au moins un compartiment 20 vide.

Par l'intermédiaire de l'unité de contrôle-commande, le robot 8 avec son organe de préhension 80 effectue alors les étapes successives suivantes :
- positionnement de la boite de transfert 2_{T} sur la table de manipulation 70 du poste de chargement/déchargement 7 des tubes à l'unité ;
- ouverture du couvercle 42 d'un des récipients de stockage 4 ;
- préhension d'une colonne de rangement 3 par la pince 81 puis son extraction du récipient de stockage 4 ;
- positionnement de la colonne de rangement 3 maintenue par la pince 81 sur le poste de chargement/déchargement 6 ;
- le cas échéant, lecture du moyen d'identification de la colonne positionnée 3, typiquement par lecture de code à barres ;
- extraction de la boite sélectionnée 2 de la colonne 3 par le vérin d'extraction 61 jusqu'à un positionnement sur la table d'attente 60 accessible par la pince 81, la colonne 3 étant toujours maintenue par la pince 81 ;
- le cas échéant, lecture du moyen d'identification de la boite sélectionnée 2, typiquement par lecture de code à barres ;
- rangement de la colonne de rangement 3 dans le récipient 4;
- fermeture du récipient 4;
- positionnement de la boite sélectionnée 2 sur la table de manipulation 70 ;
- le cas échéant, préhension des couvercles 21 de la boite de transfert 2_{T} et de la boite sélectionnée 2 par la pince 81 puis leur positionnement dans le poste 7 ;
- préhension depuis la boite sélectionnée 2 d'un ou plusieurs tubes d'échantillons 10 par l'élément de préhension 82 de la pince, et son (leur) positionnement dans la boite de transfert 2_{T} ;
- le cas échéant, lecture du moyen d'identification du ou des tubes d'échantillons sélectionnés 10, typiquement par lecture de code à barres ;
- le cas échéant, préhension des couvercles 21 depuis leur position dans le poste 7 et leur repositionnement sur le fond de la boite de transfert 2_{T} et de la boite de rangement sélectionnée 2 ;
- préhension de la boite sélectionnée 2 par la pince 81 depuis sa position sur la table de manipulation 70 et son positionnement sur la table d'attente 70 ;
- ouverture du couvercle 42 d'un des récipients de stockage 4 ;
- préhension d'une colonne de rangement 3 par la pince 81 puis son extraction du récipient de stockage 4 ;
- réinsertion de ladite boite 2 dans la colonne 3 au moyen du vérin de poussée 62 ;
- le cas échéant, préhension d'une autre boite sélectionnée d'une autre colonne du même récipient 4 ou d'un autre récipient et transfert d'autres tubes d'échantillons contenus dans l'autre boite ;
- fermeture du couvercle 42 du récipient 4.

L'opérateur récupère ensuite la boite de transfert 2T remplie avec le ou les tubes d'échantillons sélectionnés 10 depuis l'accès au poste de transfert 5.

### 2/ Stockage

Un opérateur sélectionne, par l'intermédiaire de l'interface homme-machine le dépôt d'un tube d'échantillons 10.

L'unité de contrôle vérifie alors dans sa base de données que des emplacements dans une colonne 3 et dans une boite de rangement 2 sont disponibles.

Si la réponse est positive, l'opérateur dépose alors dans le sas ou le tiroir étanche d'accès 90, une boite de transfert 2_{T} à au moins un compartiment 20 vide.

Par l'intermédiaire de l'unité de contrôle-commande, le robot 8 avec son organe de préhension 80 effectue alors les étapes successives suivantes :
- positionnement de la boite de transfert 2_{T} sur la table de manipulation 70 du poste de chargement/déchargement 7 des tubes à l'unité ;
- ouverture du couvercle 42 d'un des récipients de stockage 4 ;
- préhension d'une colonne de rangement 3 par la pince 81 puis son extraction du récipient de stockage 4 ;
- positionnement de la colonne de rangement 3 maintenue par la pince 81 sur le poste de chargement/déchargement 6 ;
- le cas échéant, lecture du moyen d'identification de la colonne positionnée 3, typiquement par lecture de code à barres ;
- extraction de la boite sélectionnée 2 à compartiment(s) 20 libre(s), de la colonne 3 par le vérin d'extraction 61 jusqu'à un positionnement sur la table d'attente 60 accessible par la pince 81, la colonne 3 étant toujours maintenue par la pince 81 ;
- le cas échéant, lecture du moyen d'identification de la boite sélectionnée 2, typiquement par lecture de code à barres ;
- rangement de la colonne de rangement 3 dans le récipient 4 ;
- fermeture du récipient 4 ;
- positionnement de la boite sélectionnée 2 sur la table de manipulation 70 ;
- le cas échéant, préhension des couvercles 21 de la boite de transfert 2T et de la boite de rangement sélectionnée 2 par la pince 81 puis leur positionnement dans le poste 7 ;
- préhension depuis la boite de transfert 2T d'un ou plusieurs tubes d'échantillons 10 par l'élément de préhension 82 de la pince, et son (leur) positionnement dans la boite sélectionnée 2 ;
- le cas échéant, lecture du moyen d'identification du ou des tubes d'échantillons sélectionnés 10, typiquement par lecture de code à barres ;
- le cas échéant, préhension des couvercles 21 depuis leur position dans le poste 7 et leur repositionnement sur le fond de la boite de transfert 2T et de la boite de rangement sélectionnée 2 ;
- préhension de la boite sélectionnée 2 par la pince 81 depuis sa position sur la table de manipulation 70 et son positionnement sur la table d'attente 70;
- ouverture du couvercle 42 d'un des récipients de stockage 4 ;
- préhension d'une colonne de rangement 3 par la pince 81 puis son extraction du récipient de stockage 4 ;
- réinsertion de ladite boite 2 dans la colonne 3 au moyen du vérin de poussée 62 ;
- le cas échéant, préhension d'une autre boite de la même colonne et transfert de nouveaux échantillons le cas échéant, préhension d'une autre boite sélectionnée de la même colonne et transfert d'autres tubes d'échantillons 10, contenus dans l'autre boite;
- préhension de la colonne de rangement 3 par la pince 81 puis sa réinsertion à l'intérieur du récipient de stockage 4;
- le cas échéant, préhension d'une autre colonne du même récipient ou d'un autre récipient et transfert et transfert d'autres tubes d'échantillons 10, contenus dans l'autre boite;
- fermeture du couvercle 42 du récipient 4.

L'opérateur récupère ensuite la boite de transfert 2T vide depuis l'accès au poste de transfert 5.

De manière générale, l'installation selon l'invention peut être mise en œuvre pour la manipulation et le stockage d'échantillons biologiques à très basses températures contenus dans des conteneurs divers et variés.

D'autres variantes et avantages de l'invention peuvent être réalisés sans pour autant sortir du cadre de l'invention.

En particulier, si dans le mode de réalisation illustré, il est prévu un seul robot 8 à six-axes, on peut très bien envisager deux robots, chacun à trois degrés de liberté indépendant de l'autre robot.

L'invention n'est pas limitée aux exemples qui viennent d'être décrits. L'invention est définie dans les revendications.

## Revendications

1. Installation (1) de manipulation et de stockage d'échantillons biologiques à très basses températures, notamment dans des conditions cryogéniques, comprenant :
- une pluralité de boites (2) de rangement à compartiments multiples, chaque compartiment (20) étant adapté pour loger un conteneur (10) adapté chacun pour contenir un ou des échantillons biologiques ;
- une pluralité de colonnes de rangement (3), chacune s'étendant selon un axe longitudinal et divisée en une pluralité de tiroirs (30) de rangement, dits racks, adaptés chacun pour recevoir par translation transversale à l'axe longitudinal une de la pluralité des boites ;
- au moins un récipient de stockage (4), à isolation thermique, dont l'intérieur est apte à être soumis à de très basses températures, comprenant dans sa partie supérieure une grille alvéolée (40) dont chaque alvéole (41) est adaptée pour recevoir à la verticale une de la pluralité des colonnes;
- un poste de transfert (5) comprenant une table de transfert (50) sur laquelle un opérateur peut amener une ou plusieurs boite(s) à compartiments multiples, dite boite de transfert (2_{T}), dont chaque compartiment (20_{T}) est également adapté pour loger un d'une pluralité de conteneurs adaptés chacun pour contenir un ou des échantillons biologiques;
- un poste de chargement/déchargement (6) des boites de rangement à l'unité, comprenant une table (60), dite table d'attente, au moins un moyen d'extraction (61) pour extraire n'importe laquelle des boites de rangement, depuis un rack d'une colonne positionnée dans ledit poste et l'amener jusqu'à une position posée sur la table d'attente et au moins un moyen de poussée (62) pour pousser une boite, depuis sa position posée sur la table d'attente, jusque dans un rack vide d'une colonne positionnée dans ledit poste ;
- un poste de chargement/déchargement (7) des conteneurs à l'unité, comprenant une table (70), dite table de manipulation, adaptée pour supporter au moins deux boites à compartiments, dont l'une est la boite de transfert et l'autre est l'une des boites de rangement sélectionnée;
au moins un robot (8), comprenant un organe de préhension (80, 81, 82) adapté pour réaliser la préhension soit de l'extrémité supérieure d'une colonne de rangement (3), soit d'une boite de rangement (2), soit d'un conteneur (10), le(s) robot(s) étant adapté(s) pour permettre un mouvement du ou des organes de préhension selon six degrés de liberté ; l'installation étant configurée de sorte que l'organe de préhension (80, 81, 82) du robot (8) puisse à la fois :
i/ amener la boite de transfert (20_{T}) depuis la table de transfert (50) sur la table de manipulation (70),
ii/ extraire n'importe laquelle des colonnes (3) depuis une des alvéoles de la grille (40) du récipient de stockage puis l'amener dans le poste de chargement/déchargement des boites (6) dans une position telle que le moyen d'extraction (61) puisse extraire une boite de rangement sélectionnée (20) sur la table d'attente (60),
iii/ amener la boite de rangement sélectionnée (20) depuis la table d'attente sur la table de manipulation (70),
iv/ extraire à l'unité n'importe lequel des conteneurs (10) depuis un des compartiments (20) de la boite de rangement sélectionnée, positionnée sur la table de manipulation (70) et l'amener dans un des compartiments (20) de la boite de transfert (20_{T}),
et vice-versa avec le moyen de poussée (62) pour pousser une boite de rangement sélectionnée (20) dans un rack vide d'une colonne positionnée dans le poste de chargement/déchargement des boites (6).

2. Installation (1) selon la revendication 1, comprenant un seul robot (8) à six degrés de liberté de mouvement, dit robot six axes.

3. Installation (1) selon la revendication 1 ou 2, comprenant une enceinte (9) à environnement contrôlé comprenant un accès sécurisé au poste de transfert pour un opérateur depuis l'extérieur, l'enceinte étant configurée pour loger la pluralité des boites de rangement (2), la pluralité des colonnes de rangement (3), le(s) récipient(s) de stockage (4), le poste de transfert (5), les postes de chargement/déchargement (6, 7) et le robot six axes (8).

4. Installation (1) selon la revendication 3, l'accès au poste de transfert consistant en un sas étanche ou en un tiroir étanche (90).

5. Installation (1) selon l'une des revendications précédentes, chaque colonne et/ou chaque boite de rangement et/ou la boite de transfert comprenant un moyen d'identification, de préférence un code à barres, par un lecteur d'identification porté par le robot.

6. Installation (1) selon l'une des revendications précédentes, comprenant une pluralité de récipients de stockage (4) agencés en formant sensiblement une portion de cercle, le robot étant fixé et agencé à l'intérieur de la portion de cercle, le poste de chargement/déchargement des boites à l'unité étant agencé à l'intérieur du cercle, le poste de chargement/déchargement des conteneurs à l'unité étant agencé sur le cercle.

7. Installation (1) selon l'une des revendications 1 à 5, comprenant une pluralité de récipients de stockage (4) agencés en formant sensiblement au moins une rangée, le robot étant monté sur un chariot mobile sur un rail le long de la rangée.

8. Installation selon l'une des revendications précédentes, le ou les récipients de stockage comprenant chacun un couvercle (42) pour fermer le récipient ainsi en configuration de stockage, non concerné par l'extraction depuis ou réciproquement l'insertion d'une colonne en son sein.

9. Installation selon la revendication 8, l'unité de contrôle-commande du robot (8) étant adaptée pour commander l'ouverture et réciproquement la fermeture de chaque récipient de stockage (4).

10. Installation selon l'une des revendications précédentes, comprenant un ou plusieurs vérins (61, 62), en tant que moyen(s) d'extraction et/ou en tant que moyen(s) de poussée, de préférence, comprenant en tant que moyens d'extraction, plusieurs vérins (61) positionnés chacun en regard d'un rack (30) d'une colonne de rangement (3) lorsqu'elle est positionnée sur la table d'attente, de préférence encore, l'organe de préhension (80, 81) du robot (8) constituant le moyen de poussée.

11. Installation selon l'une des revendications précédentes, les boites de rangement et de transfert étant adaptées pour loger des tubes de conservation (10) ou des enveloppes souples, telles que des poches de sang en tant que conteneurs d'échantillons biologiques.

12. Installation selon l'une des revendications précédentes, les boites de rangement comprenant chacune un couvercle, l'organe de préhension du robot étant alors adapté pour ôter un couvercle de sa boite avant l'extraction à l'unité des conteneurs logés dans ladite boite, et vice-versa.

13. Installation selon l'une des revendications précédentes, le ou les récipients de stockage fonctionnant à des conditions cryogéniques, notamment par azote liquide en tant que source de froid.

14. Installation selon l'une des revendications précédentes, le poste de chargement/déchargement (7) des conteneurs à l'unité étant configuré en enceinte étanche à environnement à de très basses températures.

15. Utilisation de l'installation (1) selon l'une des revendications précédentes, pour stocker des échantillons biologiques dans des conditions cryogéniques sous enceinte à environnement contrôlé.

## Patentansprüche

1. Anlage (1) zur Handhabung und Lagerung von biologischen Proben bei sehr niedrigen Temperaturen, insbesondere unter kryogenen Bedingungen, umfassend:
- eine Vielzahl von Aufbewahrungsboxen (2) mit mehreren Fächern, wobei jedes Fach (20) geeignet ist, ein Behältnis (10) aufzunehmen, das jeweils geeignet ist, eine oder mehrere biologische Proben zu enthalten;
- eine Vielzahl von Aufbewahrungssäulen (3), von denen sich jede entlang einer Längsachse erstreckt und in eine Vielzahl von Aufbewahrungsschubladen (30), Racks genannt, unterteilt ist, von denen jede geeignet ist, durch Translation quer zur Längsachse eine der Vielzahl von Boxen aufzunehmen;
- mindestens einen Lagerbehälter (4) mit thermischer Isolierung, dessen Innenraum geeignet ist, sehr niedrigen Temperaturen ausgesetzt zu werden, umfassend in seinem oberen Teil ein Zellengitter (40), von dem jede Zelle (41) geeignet ist, in der Vertikalen eine der Vielzahl von Säulen aufzunehmen;
- eine Übergabestation (5), umfassend einen Übergabetisch (50), auf den ein Bediener eine oder mehrere Boxen mit mehreren Fächern, Übergabebox (2_{T}) genannt, bringen kann, von denen jedes Fach (20_{T}) ebenfalls geeignet ist, eines einer Vielzahl von Behältnissen aufzunehmen, von denen jedes geeignet ist, eine oder mehrere biologische Proben zu enthalten;
- eine Aufgabe-/Entnahmestation zum einzelnen Aufgeben und Entnehmen (6) der Aufbewahrungsboxen, umfassend einen Tisch (60), Wartetisch genannt, mindestens ein Ausziehmittel (61), um eine beliebige der Aufbewahrungsboxen aus einem Rack einer in der Station angeordneten Säule zu ziehen und sie bis in eine auf dem Wartetisch aufliegende Position zu bringen, und mindestens ein Schiebemittel (62), um eine Box aus ihrer auf dem Wartetisch aufliegenden Position bis in ein leeres Rack einer in der Station angeordneten Säule zu schieben;
- eine Aufgabe-/Entnahmestation (7) zum einzelnen Aufgeben und Entnehmen der Behältnisse, umfassend einen Tisch (70), Handhabungstisch genannt, der geeignet ist, mindestens zwei Boxen mit Fächern zu tragen, von denen eine die Übergabebox ist und die andere eine der ausgewählten Aufbewahrungsboxen ist;
mindestens einen Roboter (8), umfassend ein Greiforgan (80, 81, 82), das geeignet ist, das Greifen entweder des oberen Endes einer Aufbewahrungssäule (3) oder einer Aufbewahrungsbox (2) oder eines Behältnisses (10) auszuführen, wobei der/die Roboter geeignet ist/sind, ein Bewegen des oder der Greiforgane gemäß sechs Freiheitsgraden zu ermöglichen, wobei die Anlage so ausgestaltet ist, dass das Greiforgan (80, 81, 82) des Roboters (8) zugleich:
i/ die Übergabebox (20_{T}) vom Übergabetisch (50) auf den Handhabungstisch (70) bringen kann,
ii/ jede beliebige der Säulen (3) aus einer der Zellen des Gitters (40) des Lagerbehälters ziehen und sie anschließend in die Aufgabe-/Entnahmestation für die Boxen (6) in eine solche Position bringen kann, dass das Ausziehmittel (61) eine ausgewählte Aufbewahrungsbox (20) auf den Wartetisch (60) ziehen kann,
iii/ die ausgewählte Aufbewahrungsbox (20) vom Wartetisch auf den Handhabungstisch (70) bringen kann,
iv/ jedes beliebige der Behältnisse (10) einzeln aus einem der Fächer (20) der ausgewählten Aufbewahrungsbox, die auf dem Handhabungstisch (70) angeordnet ist, ziehen kann und sie in eines der Fächer (20) der Übergabebox (20_{T}) bringen kann,
und umgekehrt mit dem Schiebemittel (62), um eine ausgewählte Aufbewahrungsbox (20) in ein leeres Rack einer Säule zu schieben, die in der Aufgabe-/Entnahmestation für die Boxen (6) angeordnet ist.

2. Anlage (1) nach Anspruch 1, umfassend einen einzigen Roboter (8) mit sechs Bewegungsfreiheitsgraden, Sechs-Achsen-Roboter genannt.

3. Anlage (1) nach Anspruch 1 oder 2, umfassend einen Raum (9) mit kontrollierter Umgebung, der einen gesicherten Zugang von außen für einen Bediener zur Übergabestation umfasst, wobei der Raum dazu ausgestaltet ist, die Vielzahl der Aufbewahrungsboxen (2), die Vielzahl der Aufbewahrungssäulen (3), den/die Lagerbehälter (4), die Übergabestation (5), die Aufgabe-/Entnahmestationen (6, 7) und den Sechs-Achsen-Roboter (8) aufzunehmen.

4. Anlage (1) nach Anspruch 3, wobei der Zugang zur Übergabestation in einer dichten Schleuse oder in einer dichten Schublade (90) besteht.

5. Anlage (1) nach einem der vorhergehenden Ansprüche, bei der jede Säule und/oder jede Aufbewahrungsbox und/oder die Übergabebox ein Identifizierungsmittel, bevorzugt einen Strichcode, zur Identifizierung durch ein vom Roboter mitgeführtes Identifizierungslesegerät umfasst.

6. Anlage (1) nach einem der vorhergehenden Ansprüche, umfassend eine Vielzahl von Lagerbehältern (4), die so angeordnet sind, dass sie im Wesentlichen einen Kreisabschnitt bilden, wobei der Roboter im Innern des Kreisabschnitts befestigt und angeordnet ist, wobei die Aufgabe-/Entnahmestation zum einzelnen Aufgeben und Entnehmen der Boxen im Innern des Kreises angeordnet ist, wobei die Aufgabe-/Entnahmestation zum einzelnen Aufgeben/Entnehmen der Behältnisse auf dem Kreis angeordnet ist.

7. Anlage (1) nach einem der Ansprüche 1 bis 5, umfassend eine Vielzahl von Lagerbehältern (4), die so angeordnet sind, dass sie im Wesentlichen mindestens eine Reihe bilden, wobei der Roboter auf einem Wagen montiert ist, der auf einer Schiene entlang der Reihe verfahrbar ist.

8. Anlage nach einem der vorhergehenden Ansprüche, wobei der oder die Lagerbehälter jeweils einen Deckel (42) zum Verschließen des Behälters umfassen, der somit in einer Lageranordnung ist, in der er vom Herausziehen oder Hineinführen einer Säule aus ihm bzw. in ihn nicht betroffen ist.

9. Anlage nach Anspruch 8, wobei die Steuerungseinheit des Roboters (8) geeignet ist, das Öffnen und umgekehrt das Verschließen jedes Lagerbehälters (4) zu steuern.

10. Anlage nach einem der vorhergehenden Ansprüche, umfassend einen oder mehrere Hubzylinder (61, 62) als Ausziehmittel und/oder als Schiebemittel, umfassend bevorzugt als Ausziehmittel mehrere Hubzylinder (61), die jeweils gegenüber einem Rack (30) einer Aufbewahrungssäule (3) angeordnet sind, wenn sie auf dem Wartetisch angeordnet ist, wobei noch bevorzugter das Greiforgan (80, 81) des Roboters (8) das Schiebemittel darstellt.

11. Anlage nach einem der vorhergehenden Ansprüche, wobei die Aufbewahrungs- und Übergabeboxen geeignet sind, Aufbewahrungsröhrchen (10) oder flexible Hüllen wie Blutbeutel als Behältnisse für biologische Proben aufzunehmen.

12. Anlage nach einem der vorhergehenden Ansprüche, wobei die Aufbewahrungsboxen jeweils einen Deckel umfassen, wobei das Greiforgan des Roboters dann geeignet ist, vor dem einzelnen Herausziehen der in der Box aufgenommenen Behältnisse einen Deckel von seiner Box abzunehmen und umgekehrt.

13. Anlage nach einem der vorhergehenden Ansprüche, wobei der oder die Lagerbehälter bei kryogenen Bedingungen funktionieren, insbesondere mit flüssigem Stickstoff als Kältequelle.

14. Anlage nach einem der vorhergehenden Ansprüche, wobei die Aufgabe-/Entnahmestation (7) zum einzelnen Aufgeben/Entnehmen der Behältnisse als dichter Raum mit einer Umgebung mit sehr niedrigen Temperaturen ausgestaltet ist.

15. Verwendung der Anlage (1) nach einem der vorhergehenden Ansprüche zum Lagern der biologischen Proben unter kryogenen Bedingungen in einem Raum mit kontrollierter Umgebung.

## Claims

1. An installation (1) for handling and storing biological samples at very low temperatures, notably under cryogenic conditions, comprising:
- a plurality of storage boxes (2) with multiple compartments, each compartment (20) being adapted to house a container (10) each adapted to contain one or more biological samples;
- a plurality of storage columns (3), each extending along a longitudinal axis and divided into a plurality of storage drawers (30), termed racks, each adapted to receive by translation transversely to the longitudinal axis one of the plurality of boxes;
- at least one storage receptacle (4), with thermal insulation, the interior of which is adapted to be subjected to very low temperatures, comprising in its upper part a cellular grid (40) in which each cell (41) is adapted to receive vertically one of the plurality of columns;
- a transfer station (5) comprising a transfer table (50) on which an operative can place one or more boxes with multiple compartments, termed transfer boxes (2_{T}), each compartment (20_{T}) of which is also adapted to house one of a plurality of containers each adapted to contain one or more biological samples;
- a station (6) for loading/unloading the storage boxes one by one, comprising a table (60), termed a waiting table, at least one extraction means (61) for extracting any of the storage boxes, from a rack of a column position in said station, and moving it to a position placed on the waiting table, and at least one pusher means (62) for pushing a box, from its position placed on the waiting table, into an empty rack of a column positioned in said station;
- a station (7) for loading/unloading the containers one by one, comprising a table (70), termed a handling table, adapted to support at least two boxes with compartments, one of which is the transfer box and the other of which is a selected one of the storage boxes;
- at least one robot (8), comprising a gripping member (80, 81, 82) adapted to grip the upper end of a storage column (3), or of a storage box (2), or of a container (10), the robot(s) being adapted to enable movement of the gripping member or members with six degrees of freedom;
the installation being configured so that the gripping member (80, 81, 82) of the robot (8) can simultaneously:
i/ move the transfer box (20_{T}) from the transfer table (50) onto the handling table (70),
ii/ extract any of the columns (3) from one of the cells of the grid (40) of the storage receptacle and then move it into the station (6) for loading/unloading the boxes in a position such that the extraction means (61) can extract a selected storage box (20) on the waiting table (60),
iii/ move the selected storage box (20) from the waiting table onto the handling table (70),
iv/ extract any of the receptacles (10) one by one from one of the compartments (20) of the selected storage box, positioned on the handling table (70) and move it into one of the compartments (20) of the transfer box (20_{T}),
and vice versa with the pusher means (62) to push a selected storage box (20) into an empty rack of a column positioned in the station (6) for loading/unloading the boxes.

2. The installation (1) as claimed in claim 1, comprising a single robot (8) with six degrees of freedom of movement, termed a six-axis robot.

3. The installation (1) as claimed in claim 1 or 2, comprising a controlled environment enclosure (9) providing safe access to the transfer station for an operative from the exterior, the enclosure being configured to house the plurality of storage boxes (2), the plurality of storage columns (3), the storage receptacle(s) (4), the transfer station (5), the loading/unloading stations (6, 7) and the six-axis robot (8).

4. The installation (1) as claimed in claim 3, the access to the transfer station consisting in an airtight airlock or in an airtight drawer (90).

5. The installation (1) as claimed in any one of the preceding claims, each storage column and/or each storage box and/or the transfer box comprising a means, preferably a barcode, of identification by an identification reader carried by the robot.

6. The installation (1) as claimed in any one of the preceding claims, comprising a plurality of storage receptacles (4) arranged substantially to form a portion of a circle with the robot fixed and arranged within the circle portion, the station for loading/unloading the boxes one by one being arranged inside the circle, the station for loading/unloading the containers one by one being arranged on the circle.

7. The installation (1) as claimed in any one of claims 1 to 5, comprising a plurality of storage receptacles (4) arranged substantially to form at least one row with the robot mounted on a carriage mobile on a rail along the row.

8. The installation as claimed in any one of the preceding claims, the storage receptacle or receptacles each comprising a lid (42) for closing the receptacle, not involved in the storage configuration in the extraction from it or reciprocally in the insertion in it of a column.

9. The installation as claimed in claim 8, the control and command unit of the robot (8) being adapted to command the opening and reciprocally the closing of each storage receptacle (4).

10. The installation as claimed in any one of the preceding claims, comprising one or more cylinders (61, 62), as extraction means and/or pusher means, preferably comprising as extraction means a plurality of cylinders (61) each positioned facing a rack (30) of a storage column (3) when it is positioned on the waiting table, more preferably the gripping member (80, 81) of the robot (8) constituting the pusher means.

11. The installation as claimed in any one of the preceding claims, the storage and transfer boxes being adapted to house conservation tubes (10) or flexible envelopes, such as sachets of blood as biological sample receptacles.

12. The installation as claimed in any one of the preceding claims, the storage boxes each comprising a lid, the handling member of the robot then being adapted to remove a lid from its box before the one by one extraction of the receptacles housed in said box, and vice versa.

13. The installation as claimed in any one of the preceding claims, the storage receptacle or receptacles operating under cryogenic conditions, notably with liquid nitrogen as the cold source.

14. The installation as claimed in any one of the preceding claims, the station (7) for loading/unloading the containers one by one being configured as an airtight enclosure with an environment at very low temperatures.

15. Use of the installation (1) as claimed in any one of the preceding claims to store biological samples under cryogenic conditions in controlled environment receptacles.
